# EUROPEAN PATENT APPLICATION

(11) **EP 3 698 705 A1**
(43) Date of publication of application: **26.08.2020**
(21) Application number: 19158571.0
(22) Date of filing: 21.02.2019
(51) Int. Cl.: A61B 5/00, A61B 5/01, A61B 5/024, A61B 5/0402, A61B 5/0476, A61B 5/0488, A61B 5/0496, A61B 5/053, A61B 5/08, A61B 5/11, A61M 21/00

(54) **DEVICE, SYSTEM AND METHOD FOR MANIPULATING A USER´S SLEEP STATE**

(71) Applicant: Koninklijke Philips N.V., 5656 AG Eindhoven (NL)
(72) Inventor: SHI, Jun, 5656 AE Eindhoven (NL); CHEN, Shuang, 5656 AE Eindhoven (NL); HILBIG, Rainer, 5656 AE Eindhoven (NL); BULUT, Murtaza, 5656 AE Eindhoven (NL); VAN DER ZAAN-LANDWEHR JOHAN, Suzanne Daniëlle, 5656 AE Eindhoven (NL)
(74) Representative: de Haan, Poul Erik

(57) **Abstract**

The present invention relates to a method and a device for generating a manipulation signal for manipulating a user's sleep state. The device comprises a sensor data input configured to obtain sleep-related data of a first user and a second user, an analysis unit configured to determine one or more sleep parameters of the first user and the second user based on the sleep-related data, and a manipulation signal generation unit configured to generate a manipulation signal for manipulating a sleep state of at least the second user based on the determined one or more sleep parameters of the first user and the second user. The present invention further relates to a system for manipulating a user's sleep state.

## Description

### FIELD OF THE INVENTION

The present invention relates to a device and method for generating a manipulation signal for manipulating a user's sleep state. The present invention further relates to a system for manipulating a user's sleep state.

### BACKGROUND OF THE INVENTION

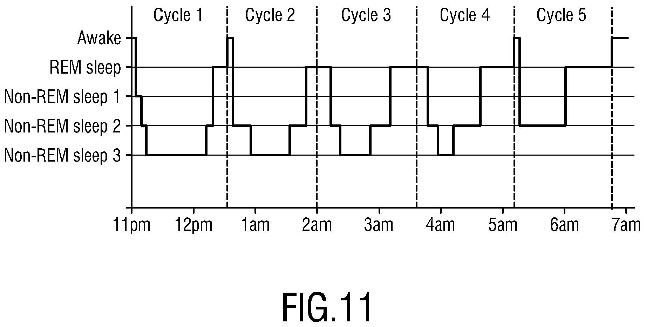

Easy wakeup is all about timing. In general, people go through different sleep state cycles during sleep. In particular, sleep progresses in a series of four or five more or less regular sleep cycles of non-REM and REM sleep throughout the night. Each cycle follows the stages of non-REM sleep (stage 1 - stage 2 - stage 3) and then, after a period in deep stage 3 slow-wave sleep, back through the stages (stage 3 - stage 2 - stage 1). Then, instead of waking, the sleeper may enter a short period of REM sleep, before going back through the stages (stage 1 - stage 2 - stage 3) in a new cycle. A typical hypnogram showing sleep stages and cycles in adult sleep is shown in Fig. 11. However, there may exist other definitions of sleep stages in a sleep cycle.

Light sleep represents the early stages of a sleep cycle, comprising stage 1 and stage 2 of non-REM sleep, in which the brain wave activity is still relatively fast and awakening is relatively easy. Deep sleep normally comprises non-REM stage 3, during which the sleeper is largely unaware of and unresponsive to the outside environment. Deep sleep is also called slow wave sleep.

Each sleep stage in any particular sleep cycle fulfills a distinct physiological and neurological function, each of which appears to be necessary for the health of the body and mind, to the extent that, if sleep is interrupted or if certain stages are missing for any reason, their physiological functions are not fully executed, and the person may feel tired or groggy even after an apparently sufficient sleep period, a phenomenon known as "sleep inertia". In fact, it is easy to wake up somebody in a light sleep or awake state, while difficult in a deep sleep state. Therefore, people may feel tired when they wake up from the deep sleep state.

Cycles of deep and light sleep last 30 to 50 minutes in babies and then gradually increase in length across childhood. For adults, the sleep cycle of deep and light sleep lasts about 90 minutes. Some babies and children fall deeply asleep very quickly while others sleep lightly for up to 20 minutes before getting into deep sleep. Children usually wake up briefly at the end of each sleep cycle. This is a normal part of healthy sleep and most children do it. Some children call out when they wake and need help setting again, but some not and put themselves back to sleep independently. Not all parents hear their children when they wake up.

There exist situations where couples may want to get up without disturbing the other sleep partner. If the sleep partner is in deep sleep, there will be least interference. However, if the sleep partner is in light sleep, he/she may probably be awakened or disturbed. In other situations, different family members may want to wake up together at a pre-set wakeup time. However, finding an optimized timing for alarm may not work all the time if the pre-set time window is short, e.g. 5 minutes. This applies even if sleep states are monitored. Hence, there may be found no suitable timing for family members to wake up easily in this short range. This may be the case, for example, for a mother who needs to wake up during the night to feed or take care of her baby.

### SUMMARY OF THE INVENTION

It is an object of the present invention to provide a device, system and method allowing users to have restful sleep even in the presence of a sleep partner or other disruptive factors.

In a first aspect of the present invention a device for generating a manipulation signal for manipulating a user's sleep state is presented, the device comprising:
- a sensor data input configured to obtain sleep-related data of a first user and a second user,
- an analysis unit configured to determine one or more sleep parameters of the first user and the second user based on the sleep-related data, and
- a manipulation signal generation unit configured to generate a manipulation signal for manipulating a sleep state of at least the second user based on the determined one or more sleep parameters of the first user and the second user.

In a second aspect of the present invention a system for manipulating a user's sleep state is presented, the system comprising:
- a tracking unit configured to acquire sleep-related data of a first user and a second user by tracking sensor data of the first user and the second user,
- a manipulation unit configured to obtain a manipulation signal and to manipulate a sleep state of at least the second user based on the manipulation signal, and
- a device for generating a manipulation signal for manipulating a user's sleep state as disclosed herein.

In yet further aspects of the present invention, there are provided a corresponding method, a computer program which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed system, method, computer program and medium have similar and/or identical preferred embodiments as the claimed device, in particular as defined in the dependent claims and as disclosed herein.

The present invention is based on the idea to provide a device, system and method allowing a user to get restful sleep even in the presence of a sleeping partner or a baby who needs to be taken care of during the night, for example. Furthermore, the present invention shall allow users to get restful sleep when the sleeping time is limited.

Current devices used to manipulate sleep allow a user to wake up in a desired sleep state and/or at a pre-set wake-up time. However, so far the problem of waking up rested even in the presence of sleeping partners waking up earlier or when a baby is crying during the night has not been solved.

For users wishing to be wakened in a coordinated manner, the present invention provides a way to manipulate their sleep dependently. By monitoring the sleep of at least two users, in particular by collecting sleep-related data of a first user and a second user, there may be determined one or more sleep parameters of these users. Sleep-related data may comprise any of physiological data, behavioral data and user data, wherein physiological data comprise ECG data, EEG data and respiration data, for example, and wherein behavioral data comprise a user's leg movement, for example. User data may comprise data of the user's usual sleeping times, for example. The sleep-related data may be obtained in real-time from a tracking unit or from a data carrier comprising data representing the user's sleeping history. The sleep parameters determined from the sleep-related data may comprise any of sleep efficiency, sleep latency, wake-up time (during the night), (total) sleep time and sleep state of the user, wherein a sleep state refers to a sleep stage, a sequence of sleep stages and/or a transition between sleep stages in a period of time.

Particularly, the sleep parameter may comprise a user's sleep stage, more particularly REM sleep stage, NON-REM stage 1, non-REM stage 2, non-REM stage 3 and also an awake stage. However, the sleep parameter may also comprise a time for an estimated end of a sleep stage or end of sleep.

For example, the sleep-related data may comprise information about a user's sleep cycle. With the sleep cycle information and the sleep start time and expected wake-up time of said user, the number of sleep cycles until said user's sleep ends could be estimated by the analysis unit. Generally, if more data is available, such as data comprising past sleep cycles of the first and/or the second user, the estimation may be more reliable and accurate. However, even without such personalized data, data from similar populations and users can be used to perform initial estimates.

The device further comprises a manipulation signal generation unit using the one or more sleep parameters of the at least two users to generate a manipulation signal for manipulating a sleep state of at least one of the users. This way, the device provides sleep manipulation for a user which is dependent upon the sleep situation of the at least one other user. In case the sleep of more than one user is manipulated, each of the users may receive a different manipulation signal.

In general, the manipulation signal generated is used to manipulate a sleep cycle or a sleep state at the end of sleep to become a sleep state suitable for easy wake up. Preferably, there is generated a manipulation signal by the manipulation signal generation unit which causes said sleep state to be a light sleep state or an awake state. The sleep manipulation may happen at the ending sleep cycle, i.e. at a sleep cycle estimated to be the last one of a user's sleep, or at sleep cycles before said cycle. In other words, the sleep cycle or the sleep state manipulated may be in any sleep cycle or sleep state during the sleep of the user and is not limited to the last sleep cycle or sleep state in the sleep of said user.

The device works for users situated in the same room, but may also be used for users sleeping apart in different locations. Furthermore, the device may be used by more than two users. For example, the device or several connected devices may be used to coordinate the sleep of a whole family.

In general, the device or system may be implemented in a conventional alarm clock, in a smart watch, in a smartphone, in a headband, in a sleep-mask, in a pillow, in a blanket and the like.

The method of the present invention may be implemented as a smartphone app, for example.

In an embodiment of the device, the sleep-related data comprises information about a current and/or one or more prior sleep parameters of the first user and the second user. In particular, the sleep-related data may comprise information about a current and/or one or more prior sleep states of the first and the second user. For example, the sleep related data may comprise information about the sleep states of a number of prior sleep cycles or for a predefined period of time.

In another embodiment of the device, the analysis unit is configured to estimate a current sleep state and/or one or more prior sleep states and/or to predict one or more upcoming sleep states. For example, the analysis unit may estimate a current sleep state to correspond to an REM-sleep stage and to predict that the upcoming sleep states correspond to non-REM sleep stages. In general, the role of the analysis unit is to predict the sleep stage of the users at the ending wake-up time and to decide the timing and schemes for sleep state manipulation. When the timing and schemes are decided, they are fed to the manipulation signal generation unit for corresponding actions.

In a further embodiment, the manipulation signal generation unit of the device is configured to generate the manipulation signal based on a target condition of the first and/or the second user. In case that there does not only exist a single target condition, in particular if there exist target conditions of more than one user, the manipulation signal generation unit may prioritize the conditions and generate the manipulation signal in accordance with the priority of the conditions. However, the target conditions may already comprise a priority, which has been determined by the users themselves, for example.

In an example, the device for generating a manipulation signal may be used to obtain sleep-related data of a baby and his/her mother and to determine a sleep-parameter of both these users, wherein the manipulation signal generation unit may be configured to provide a manipulation signal in order to manipulate the mother's sleep. In particular, the mother's sleep may be manipulated in accordance with a target condition of the baby, for example the estimated eating-time of the baby. Accordingly, the manipulation signal may be generated in accordance with said condition. Therefore, the mother may wake-up easily during the night to feed her baby.

In another embodiment of the device, the target condition comprises any of a target wake-up time, a target wake-up time range, particularly between a first wake-up time of the first user and a second wake-up time of the second user, a target sleep state and a target environmental condition, particularly a target illumination, a target temperature, a target air composition, a target smell, a target humidity, a target contact stimulation, a target vibration stimulation and a target sound. The target sleep state may be a particular sleep state, such as a deep sleep state. In fact, a user may wish to have longer or more deep sleep stages, for example. The target temperature may comprise a target temperature of the room or the mattress, for example. In general, the target condition may also comprise a vital sign condition relating to a vital sign of any of the first and the second user such as a body temperature, for example.

In an example, the first and second user of the device may be two people sleeping in the same environment, for example a husband and his wife sleeping in the same room, not necessarily sleeping the same bed, wherein the husband wishes to wake up at a pre-defined time without disturbing his sleeping wife. However, there are also situations conceivable, wherein the husband and his wife may wish to wake-up together in a pre-defined time range, for example between 6am and 7am, and at a pre-defined room temperature of 21°C.

Preferably, the target wake-up time comprises a time range, wherein the time range may be predetermined or adjustable. For example, the target wake-up time may be a particular time with a tolerance of a number of minutes, for example +/- 10 minutes. In other words, the time tolerance represents a time window Δt, in which users want to wake up. For example, a usual alarm clock could be set to ring at 6am. However, this wake-up time may be too sharp for two sleep stages of two different users to meet. Therefore, a tolerance could be set by the users, e.g. +/- 5 minutes. Then the alarm clock could be triggered in between 5:55am to 6:05am. If the user's preset wake-up time window still cannot be met, other (pre-) settings may be used to generate a manipulation signal.

The target wake-up time may be predetermined according to any of pre-knowledge, physiological data or behavior data. Furthermore, the target wake-up time may be automatically adjusted based on an artificial intelligence algorithm.

In still another embodiment, the manipulation signal generation unit is configured to adjust the target condition of at least the second user based on the sleep-related data and/or the determined one or more sleep parameters of the first user and/or the second user. Adjusting the wake-up time is particularly advantageous if the time for manipulating the user's sleep is not sufficient to achieve a desired sleep state. For example, in case the second user has set a particular target wake-up time but there is not enough time to manipulate the user's sleep such that he is in a light sleep state, it may be advantageous to shift the target wake-up time for some minutes to have sufficient time for an appropriate sleep manipulation.

In a further embodiment, the manipulation signal generation unit is configured to generate the manipulation signal comprising synchronization information for synchronizing at least one sleep state of the second user with at least one sleep state of the first user. In other words, the manipulation signals may comprise synchronization information for synchronizing at least one sleep state of the second user with at least one sleep state of the first user. In particular, the synchronization information may be used to synchronize a sleep stage of the first user and the second user. For example, the manipulation signal generation unit may be configured to synchronize the REM-sleep stages of the first user and the second user, particularly in accordance with a target condition such as a target wake-up time.

In yet a further embodiment of the device, the manipulation signal generation unit is configured to generate the manipulation signal comprising stimulation information for stimulating at least the second user. In other words, the manipulation signal may comprise stimulation information used to stimulate at least one of the two users. According to another embodiment, the stimulation information comprises information about changing any of temperature, air composition, smell, illumination, sound, humidity, a contact stimulation and a vibration stimulation. While the first user may be stimulated to fall deeply asleep by applying a massage, for example, the second user may be stimulated to wake up by increasing a sound level in his room.

According to a further embodiment, the manipulation signal generation unit is configured to generate the manipulation signal comprising any of shifting information for shifting a sleep state, shortening information for shortening a sleep state, extending information for extending a sleep state and transformation information for transforming a sleep state, in particular into a light sleep state or a deep sleep state.

For example, if it is desired that the first user and the second user wake up at the same time, it may be necessary to extend an REM-stage of the first user and to shorten a non-REM stage of the second user.

In still another embodiment of the device for generating a manipulation signal for manipulating a user's sleep state, the analysis unit is configured to determine the one or more sleep parameters of the first user and/or the second user based on environmental data and/or user data. Indeed, environmental conditions such as the time of the year or the weather may impact the user's sleep and accordingly respective sleep parameters. In general, environmental conditions comprise any of temperature, illumination, sound, humidity, time of the day, time of the year and the like. The same applies to user data, wherein user data comprise any of age, gender, fitness, state of health etc. of the first and/or the second user. In particular, the analysis unit may be configured to predict the one or more upcoming sleep states based on environmental data and/or user data.

In an advantageous embodiment, the manipulation signal generation unit is configured to generate the manipulation signal further based on environmental data and/or user data. For example, if a mother wishes to wake-up easily in the night for feeding her baby, there may rather be generated a manipulation signal for manipulating a mother's sleep state than a sleep state of the baby.

In a preferred embodiment, the device further comprises a user interface configured to obtain user data from the first and/or the second user. The user data may comprise any of age, gender, fitness, state of health etc. of the first and/or the second user. Particularly, the user data may comprise information about an assignment of the users to a dominant user group and a dependent user group. However, the manipulation signal generation unit may also be configured to assign the first and the second user to said groups based on the user data. Generally, the sleep states of the dominant user are selected to be followed by users of the dependent user group. For a baby that needs to be taken care of in the night and a mother as first and second users, respectively, the baby may be assigned to the dominant user group by the manipulation signal generation unit whereas the mother may be assigned to the dependent user group. Accordingly, the manipulation signal generated is configured to cause the sleep of the mother to follow the sleep of the baby. In some cases, the manipulation signal generation unit may only provide a manipulation signal for a dependent user, but not for a dominant user. This is, for example the case, for a mother who needs to feed her baby in the night. In this case, the mother may be assigned to be the dependent user and there will only be generated a manipulation signal for the mother. The dominant person's sleep, i.e. the baby's sleep in this example, will not be manipulated, and only the baby's sleep (and/or the baby's hunger will be tracked by the system.

In other cases, there may be generated a manipulation signal for all users, irrespective of their assignment to a dominant or dependent user group. If the sleep states of all users are allowed to be manipulated, it may even be possible to comply with a short wake-up time tolerance.

Generally, there exist several combinations for the relation between the sleep states of a dominant member and a dependent member, i.e. users corresponding to the dominant user group and the dependent user group, respectively, at a pre-set target wake-up time (of any of the two users):
a) both dominant and dependent member's predicted sleep states are a light sleep state or an awake state;
b) the dominant member's predicted sleep state is a light sleep state or an awake state and the dependent member's predicted sleep state is a deep sleep state;
c) the dominant member's predicted sleep state is a deep sleep state and the dependent member's predicted sleep state is a light sleep state or an awake state;
d) both dominant and dependent member's predicted sleep states are deep sleep states.

If both the dependent and the dominant user wish to wake up at the same target wake-up time, the manipulation signal generation unit may perform the following actions:
In case a) there is no need for the manipulation signal generation unit to generate a manipulation signal.
In case b), the manipulation signal generation unit may be configured to generate a manipulation signal for the dependent member in order to make waking up easier for this member. In particular, the manipulation signal generation unit may turn the deep sleep stage of the dependent member to a light sleep stage, e.g. to a non-REM stage 2 or a non-REM stage 1. The manipulation signal may be provided to a wake-up light, for example, which may be configured to manipulate the dependent user's sleep with a directional wake-up light with adjustable light intensity. However, the signal may likewise be provided to a wearable gentle vibration device or to a temperature control device configured to increase the room temperature or mattress temperature to a certain pre-set level.

However, in case b) the manipulation signal generation unit may likewise be configured to generate a manipulation signal comprising shifting information for shifting the last deep sleep stage to be combined with a previous cycle. This way, the total time for deep sleep is kept unchanged. For increasing the deep sleep quality, the manipulation signal may be provided to a temperature control device for decreasing the room temperature or mattress temperature to a certain pre-set level. However, said signal may likewise (or furthermore) comprise information to control a local CO2 level of the air surrounding the dependent member.

In case c), the manipulation signal generation unit may be configured to generate a manipulation signal for the dominant member. However, in order to manipulate the dominant member as little as possible there may be introduced a wake-up tolerance or a pre-set wake-up tolerance, i.e. a time tolerance range around the target wake-up time, may be increased. Furthermore, the manipulation signal may be generated in accordance with a prioritization of a target sleep stage and a target wake-up time. For example, if a target sleep state has been prioritized over the target wake-up time, the manipulation signal generation unit may take more time to manipulate the dominant member's sleep.

In case d), the manipulation signal generation unit may be configured to generate a manipulation signal for both the dominant and dependent member. In order to ensure that the dominant member is manipulated as little as possible, the same measures as in case c) may be taken.

If only the dependent member wishes to be wakened at the target wake-up time with the dominant member still sleeping, the manipulation signal generation unit may perform the following actions:
In case a), the manipulation signal generation unit may be configured to generate a manipulation signal for the dominant member.
In case b) the manipulation signal generation unit may be configured to generate a manipulation signal for both users.
In case c) there is no need for the manipulation signal generation unit to generate a manipulation signal.
In case d), the manipulation signal generation unit may be configured to generate a manipulation signal for the dependent member.

In an embodiment of the system for manipulating a user's sleep state, the tracking unit comprises any of an electroencephalograph, an electrocardiograph, an electrooculograph, an electromyograph, a camera, a movement sensor, an inertia sensor, a heart rate monitor, a heart rate variability monitor, a sweat rate monitor, a galvanic skin response sensor, a microphone, a pressure sensor, a respiration rate and respiration depth monitor, and a body temperature sensor. In general, the tracking unit may be configured to collect sensor data, i.e. physiological and/or behavioral data, of the first user and/or the second user. For example, the tracking unit may comprise movement sensors in a mattress of at least the second user. The sensor data particularly comprise a vital sign of the first user and the second user, wherein the vital sign may comprise any of movement, heart beat, heart rate, sweat rate, respiration rate, respiration depth, body temperature or the like.

Generally, the tracking may comprise several sensors, wherein a first sensor may be configured to acquire sleep-related data of a first user by tracking sensor data of the first user and wherein a second sensor may be configured to acquire sleep-related data of a second user by tracking sensor data of the second user. However, the first sensor may also be configured to track sensor data of the second user and vice versa. For example, the tracking unit may comprise a body temperature sensor configured to measure the body temperature of the first user and the second user and a heart rate monitor configured to measure the heart rate of the second user.

In an advantageous embodiment, the system further comprises a user interface configured to obtain user data from the first and/or the second user. User data may comprise information about any of age, gender, fitness, state of health, sleeping habits such as sleeping times etc. of the first and/or the second user. User data may likewise comprise information about the user's assignment to a dominant or dependent user group.

### BRIEF DESCRIPTION OF THE DRAWINGS

These and other aspects of the invention will be apparent from and elucidated with reference to the embodiments described hereinafter. In the following drawings
Fig. 1 shows a schematic diagram of a first embodiment of a device for generating a manipulation signal for manipulating a user's sleep state according to the present invention,
Fig. 2 shows a schematic diagram of a second embodiment of a device for generating a manipulation signal for manipulating a user's sleep state according to the present invention,
Fig. 3 shows an exemplary implementation of a device according to the present invention in the form of a wearable device,
Fig. 4 shows a schematic diagram of a first embodiment of a system for manipulating a user's sleep state according to the present invention,
Fig. 5 shows a schematic diagram of a second embodiment of a system for manipulating a user's sleep state according to the present invention,
Fig. 6 shows a schematic diagram of a third embodiment of a system for manipulating a user's sleep state according to the present invention,
Fig. 7 shows a flow chart of a first embodiment of a method for generating a manipulation signal for manipulating a user's sleep state according to the present invention,
Fig. 8 shows a flow chart of a second embodiment of a method for generating a manipulation signal for manipulating a user's sleep state according to the present invention,
Fig. 9 shows a tracking of sleep stages for two users A and B over a number of sleep cycles,
Fig. 10 shows a sequence sleep stages for two users C and D over a number of sleep cycles as predicted by the device of the present invention, and
Fig. 11 shows a typical hypnogram of sleep stages and cycles in adult sleep.

### DETAILED DESCRIPTION OF THE INVENTION

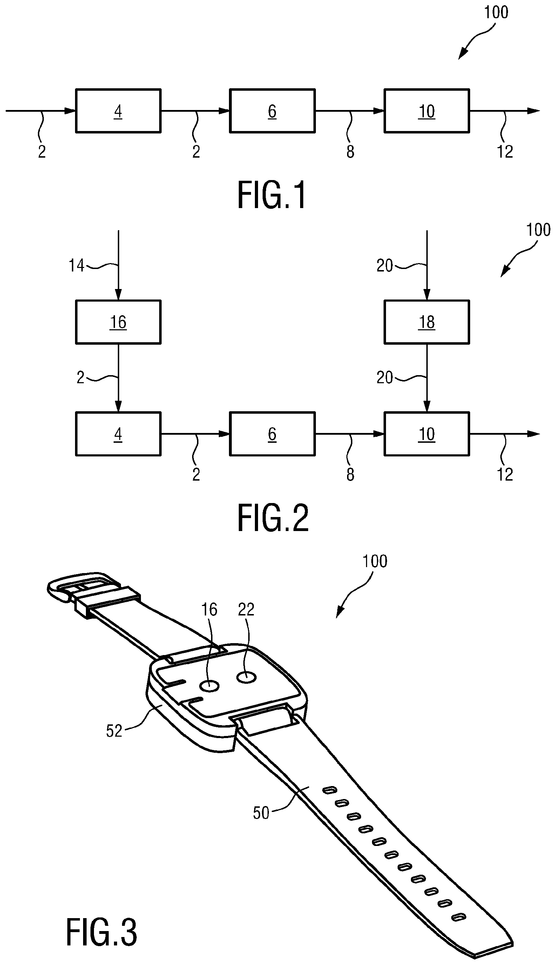

Fig. 1 shows a schematic diagram of a first embodiment of a device 100 for generating a manipulation signal for manipulating a user's sleep state. The device's sensor data input 4 is configured to obtain (retrieve or receive) sleep-related data 2 of at least a first user and a second user. The sleep-related data 2 comprises ECG data, for example. The obtained data are then provided to the analysis unit 6, which uses said data to determine one or more sleep parameters 8 of at least the first user and the second user. In this embodiment, the sleep parameters 8 particularly comprise the user's current sleep state and prior sleep states, wherein the prior sleep states are sleep states corresponding to a predefined time. The manipulation signal generation unit 10 uses the sleep parameters 8, i.e. the sleep states in this embodiment, to generate a manipulation signal 12 for manipulating an (upcoming) sleep state of at least the second user. The manipulation signal 12 may comprise a signal for informing a manipulation unit such as a light alarm clock to increase and/or decrease its illumination directed to the second user, for example.

Fig. 2 shows a schematic diagram of a second embodiment of a device 100 for generating a manipulation signal for manipulating a user's sleep state. In this embodiment, the device 100 comprises a tracking unit 16, a sensor data input 4, an analysis unit 6, a manipulation signal generation unit 10 and a user interface 18.

The tracking unit 16 may comprise an electrooculograph configured to measure the eye movement as sensor data 14 of the at least two users of the device 100. The measurement results are then provided to the sensor data input 4 as sleep-related data. The analysis unit 6 uses the measurement results to analyze the sleep of the at least two users. In particular, the analysis unit estimates the depth of the sleep of the at least two users over a predefined period of time. The estimated depth of sleep over the predefined period of time is then provided to the manipulation signal generation unit 10. In addition to this sleep parameter 8 determined by the analysis unit 6, the manipulation signal generation unit 10 is configured to use user data 20 for generating the manipulation signal 12. The user data 20 may be obtained by the user interface 18 coupled to the manipulation signal generation unit 10. The user interface may comprise any of a touchscreen, a keyboard, a speech recognition module and the like. In an example, the user data 20 may comprise information about health state and drug use of at least the second user. In fact, for a user taking sleep pills, for example, the manipulation signal 12 may comprise information for a manipulation that is stronger than for a sober user.

Fig. 3 shows an exemplary implementation of a device 100 according to the present invention in the form of a wearable device. In this embodiment, the wearable device 100 is implemented in the form of a smart watch and is configured to be worn by the second user. The smart watch comprises a wristband 50 and a casing 52. The wristband 50 can loop around the wrist of the user. It will be understood that a wearable device 100 could also be worn around another suitable part of the body such as the ankle foot or hand or may be adapted for attachment to other parts of the body, e.g. in the form of a patch.

The wearable device 100 comprises a sensor data input 4, an analysis unit 6 and a manipulation signal generation unit 10 (all of which are not shown in Fig. 3) inside the casing 52. Furthermore, in this embodiment, the device 100 comprises a tracking unit 16 configured to track a vital sign of the second user and a manipulation unit 22 configured to manipulate a sleep state of the second user. Both the tracking unit 16 and the manipulation unit 22 may be installed at a skin-facing side of the casing 52. Furthermore, the device 100 may comprise a user interface 18 on the opposite side of the casing 52.

The tracking unit 16 may comprise a skin conductance sensor, for example, configured to measure the conductance of the second user's skin. The corresponding measurements of the second user's skin conductance are provided to the sensor data input 4 as (second) sleep-related data 2a. Furthermore, the data input 4 is configured to obtain (first) sleep-related data 2b of at least one other user, for example from a sleeping partner of the second user. The analysis unit 6 uses the sleep-related data 2a and 2b of the second user and the at least one other user to determine sleep parameter 8a and 8b of the first and second user. The sleep parameters 8a and 8b may then be used by the manipulation signal generation unit 10 to generate a manipulation signal 12 for the manipulation unit 16. Apart from the sleep parameters 8a and 8b the manipulation signal generation unit 10 may also use user data 20 provided by the user interface 18 to generate the manipulation signal 12. The user data 20 may comprise information about at least the second user. In particular, the user data 20 may comprise a target condition of at least the second user, particularly a target wake-up time of the second user, i.e. a time at which the second user whishes to be awakened.

Based on the manipulation signal 16 the manipulation unit 22 may then manipulate a sleep state of the second user. In this embodiment, the manipulation unit 22 comprises a vibrating element configured to cause vibration of the device 100. Accordingly, the vibrating element may vibrate for a predefined time prior to the target wake-up time specified by the second user in order to wake up the second user at the target wake-up time.

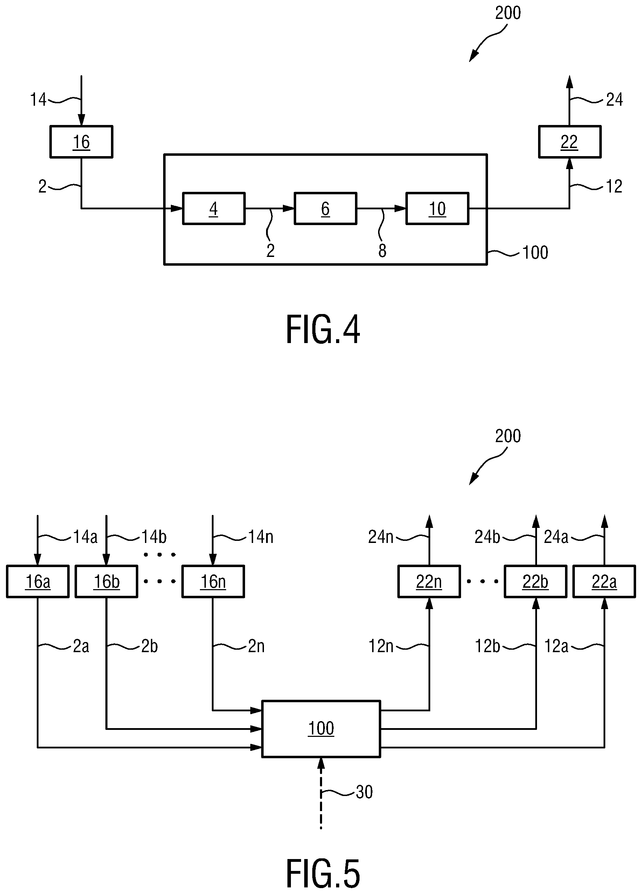

Fig. 4 shows a schematic diagram of a first embodiment of a system 200 for manipulating a user's sleep state.

The system 200 comprises a tracking unit 16, a device 100 for generating a manipulation signal for manipulating a user's sleep state and a manipulation unit 22. The device 100 of the system 200 may be located at a remote location or implemented in a remote device (e.g. a remote computer, smartphone or patient monitor).

In this embodiment, the system 200 may be used to track the sleep states of two family members and to manipulate the sleep of at least one of these two members in accordance with the sleep states measured for both family members during their sleep. Using the system 200 the family members could wake up synchronized and easily or they could wake up without disturbing each other. Using the sleep states tracked by the tracking unit 16 the analysis unit 6 is configured to estimate the current sleep state of both users, i.e. family members, and to predict one or more upcoming sleep states of the two users. Based on the analysis the manipulation unit 22 may manipulate one or both user's upcoming sleep states by performing a user stimulation 24. A target condition taken into account by the manipulation signal generation unit 10 for generating the manipulation signal may be that the target sleep states of the users at the target wake-up time are suitable for easy wake up, e.g. light sleep stage or an awake state. The user stimulation may a stimulation with directed light, for example.

Fig. 5 shows a schematic diagram of a second embodiment of a system 200 for manipulating a user's sleep state.

In this embodiment, the system 200 comprises a plurality of separate tracking units 16a to 16n configured to acquire sleep-related data of n users by tracking sensor data, particularly a vital sign, of the n users. For example, there may be tracked the different sleep states of different family members. These family members could be located in the same room or separately located in different rooms. The main function of the tracking units is to track the sleep states of individuals. Different techniques could be used to monitor the sleep states of the sleep cycles, e.g. electroencephalography, measurement of inertia movement or other movement measurements or recording a video of the user via a camera.

The device 100, particularly the input data unit 4 of the device 100, is configured to communicate with the different tracking units 16a to 16b and to collect the sleep-related data 2a to 2n correspondingly. The communication between the device 100 and the tracking units 16a to 16b may preferably be in a wireless mode.

The collected sleep-related data 2a to 2n is then transmitted to the analysis unit 6 of the device 100 for further processing. In particular, said data are used by the device for generating manipulation signals 12a to 12n for the manipulation units 22a to 22n used to manipulate the sleep states of the family members. Optionally, the device 100 may further be configured to obtain environmental data 30 for generating the manipulation signals 12a to 12n. For example, the device 100 may use weather data for generating said signals. For example, in a stormy night with lightning and thunder the manipulation signal generation unit 10 may be configured to generate a manipulation signal comprising extending information for extending deep sleep stages.

The manipulation units 22a to 22n are configured to use the manipulation signals 12a to 12n for manipulating the sleep states of the n users by performing user stimulations 24a to 24n.

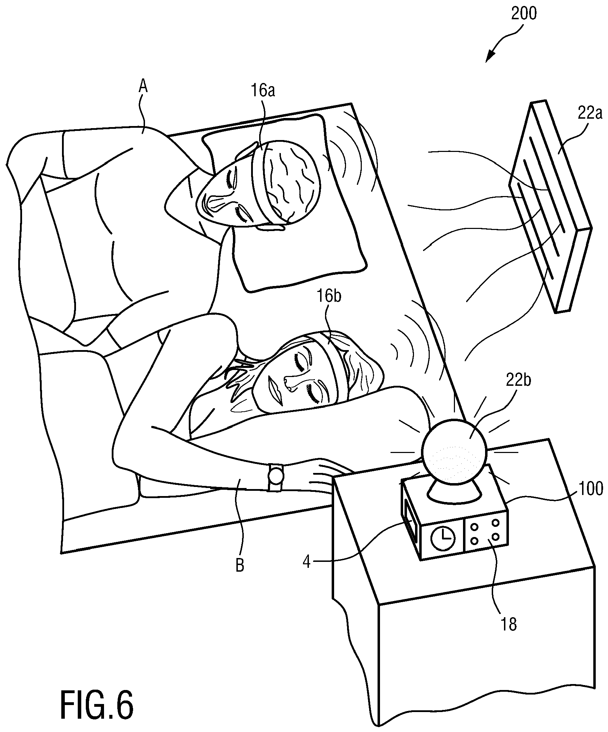

Fig. 6 shows a schematic diagram of a third embodiment of a system 200 for manipulating a user's sleep state according to the present invention. In this embodiment, the system 200 is used by two users A and B sleeping in the same bed. Both users A and B wear a headband comprising tracking units 16a and 16b, respectively. The tracking units 16a and 16b are configured to measure the pulse rate of the respective user for a pre-determined period of time. The tracking units 16a and 16b are configured to transmit the measurement results wirelessly to a device 100 for generating a manipulation signal 12 for manipulating a user's sleep state.

The device 100 may be implemented in the form of an alarm clock standing on a bedside table, for example. In particular, the tracking units 16a and 16b may transmit the measured pulse rates to the data input unit 4 of the device 100.

The analysis unit 6 and the manipulation signal generation unit 10 of the device 100 then use pulse rate data for further processing. In this embodiment, the device 100 also comprises a user interface 18 in the form of a touchscreen. Using the user interface 18 the users A and B may specify who takes the role of the dominant user and who takes the role of the dependent user, wherein the dominant user is the one who's sleep state shall be followed by the other user. Furthermore, the user data 20 provided to the user interface 18 may comprise information about a preferred wake-up time of the dependent user. With the dominant member, dependent member and target wake-up time set, the sleep state of the dominant member may be manipulated as less as possible and the sleep state of other member may be manipulated accordingly.

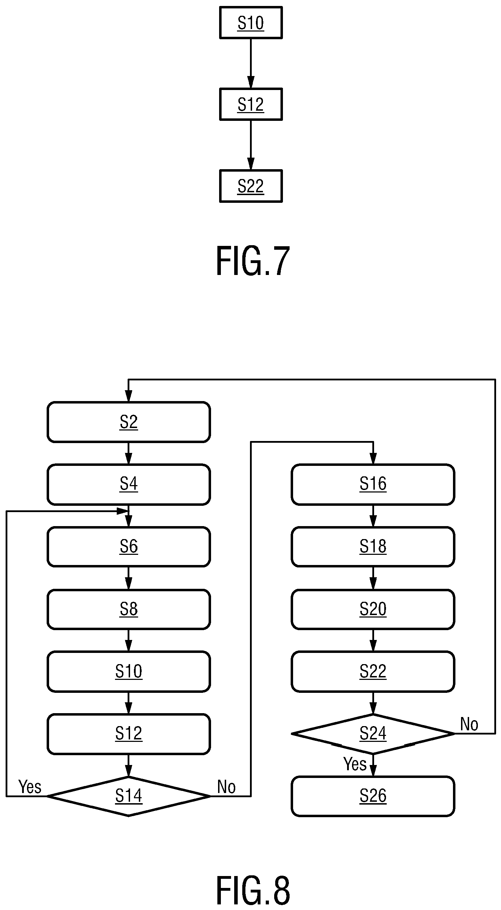

Fig. 7 shows a flow chart of a first embodiment of a method for generating a manipulation signal for manipulating a user's sleep state according to the present invention. In a first step S10, the method comprises obtaining sleep-related data of a first user and a second user, wherein the sleep-related data may comprise information about sleep states of the users, for example. A second step S12 comprises determining one or more sleep parameters of the first user and the second user based on the sleep-related data. For example, there may be determined one or more sleep states of the first user and the second user at a predefined target wake-up time. In a third step S22 there is generated a manipulation signal for manipulating a sleep state of at least the second user based on the determined one or more sleep parameters. The manipulation signal may be sent to a manipulation unit comprising a wake-up light, for example.

Fig. 8 shows a flow chart of a second embodiment of a method for generating a manipulation signal for manipulating a user's sleep state according to the present invention. In a first step S2 the method comprises acquiring the identities of the users and assigning the users to a dominant or dependent user group, respectively. In general, users who's sleep states shall be followed are assigned to the dominant user group. In a second step S4, the method comprises acquiring a target wake-up time of the users, i.e. a time at which the users wish to be wakened. Optionally, the target wake-up time may be supplemented with a wake-up time tolerance range, for example a tolerance range of five minutes around the target-wake-up time. Step 6 comprises estimating the sleep cycles of the members of the dominant user group. Step 8 comprises estimating the sleep cycles of the members of the dependent user group. Step 10 comprises obtaining sleep-related data of the users irrespective of their assignment to the dominant or dependent user group. In principle, the chronological order of steps S4, S6, S8 and S10 may be different from shown in Fig. 8.

Step 12 comprises estimating the sleep states of the users at the target-wake-up time of the users. Step 14 comprises determining whether an expected time of an awake/light sleep state matches with the preset target wake-up time (preferably, by taking into account a wake-up time tolerance). If there is a matching, the method goes back to step S6. If there is no matching, the method comprises deciding which sleep cycles of which user shall be manipulated in step S16 in accordance with the desired sleep stages at the target wake-up time. Furthermore, the method comprises deciding which sleep stage of the respective users shall be manipulated in accordance with the estimated cycle number and sleep stage at the target wake-up time in step S18. Step S20 comprises deciding the means to manipulate a particular sleep stage, i.e. deciding for an appropriate manipulation unit. This may be done in accordance with user data. In step S22, a corresponding manipulation signal is provided to a manipulation unit. In step 24, it is determined whether the target wake-up time is reached or not. If the target wake-up time is reached, the method ends. If not, the method starts over at step S2.

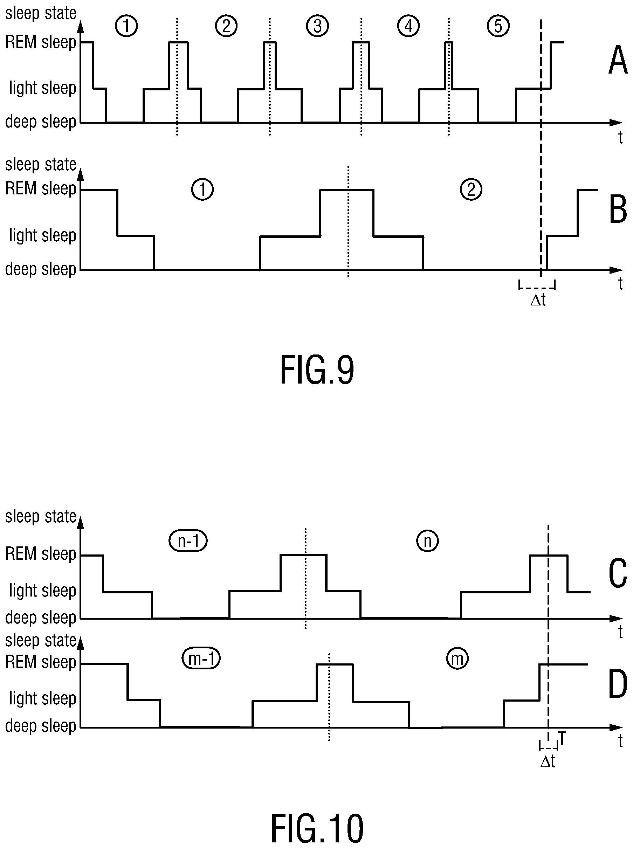

Fig. 9 shows a tracking of sleep stages for two users A and B over a number of sleep cycles. In particular, Fig. 9 shows five sleep cycles of a baby (A) taking place simultaneously with two sleep cycles of a mother (B) until a pre-set wake-up time. The sleep cycles comprise several stages. In particular, the sleep stages shown in Fig. 9 are simplified with respect to real sleep stages and comprise REM sleep, light sleep and deep sleep, wherein both light sleep and deep sleep belong to Non-REM sleep.

Waking up normally happens during light sleep or REM sleep and it is generally preferred to wake up users at these stages, in particular in a light sleep stage.

The hypnogram of Fig. 9 shows the connection of the sleep stages. In particular, it shows that a light sleep is connected with an REM sleep stage and with a deep sleep stage. However, a deep sleep stage and an REM sleep stage are generally separated from each other by a light sleep stage.

As is illustrated in Fig. 9, a sleep cycle of the mother (B) lasts longer than a sleep cycle of the baby (A). Therefore, the sleep stages of the user A and B drift apart after the onset of sleep. However, there exist also times, where the sleep stages are synchronized, for example, where both the user A (baby) and the user B (mother) are at light sleep. This is the case, for example, at the end of the first sleep cycle of the mother and at the beginning of the third sleep cycle of the baby. As can be seen from Fig. 9, at the pre-set wake-up time, marked by the dashed vertical line, the baby is in a light sleep state, whereas the mother is in a deep sleep state. Accordingly, the device for generating a manipulation signal for manipulating a user's sleep state may be configured to generate a manipulation signal for manipulating the mother's sleep stage at the pre-set wake-up time to be in a light sleep stage. If a tolerance is set by user, e.g. +/- 5 minutes, the analysis unit may analyze the sleep state of mother and predict whether a light sleep stage will happen in said tolerance time window according to the mother's sleep rhythm. If yes, no manipulation signal is generated. As can be seen from Fig. 9, the mother (B) enters a light sleep stage in the wake-up time tolerance Δt and also the baby (A) is in a light sleep stage in said tolerance time. Therefore, in this case no manipulation signal is needed.

Fig. 10 shows a sequence sleep stages for two users C and D over a number of sleep cycles as predicted by the device of the present invention. The user C and D may be a husband and his wife in this example. As can be seen from Fig. 10, it is predicted by the device that at the target wake-up time T of the husband C and the wife D are both not in a light sleep stage, but in an REM sleep stage. Furthermore, inside the time tolerance window Δt neither the user C nor the user D enters a light sleep stage. In this case, the manipulation signal generation unit may decide to manipulate both user C's and user D's sleep. As shown in Fig. 10, the cycle n for user C and cycle m for user D may be manipulated before the wake-up time T.

Given that the husband C wishes to wake up at the target wake-up time T without disturbing his sleeping wife D the manipulation signal generation unit of the device for generating a manipulation signal may generate a manipulation signal for the wife D to manipulate her sleep to be in a deep sleep stage at time T. To this end, there is measured the time between the expected end of the last deep sleep state of the wife before time T and the wake-up time T of the husband. If the manipulation signal generation unit estimates that the measured time difference can be accommodated by increasing the deep sleep at cycle m, then the manipulation signal generation unit generates a corresponding manipulation signal to increase the duration of said deep sleep stage, for example a sound stimulation signal. However, if the manipulation signal generation unit estimates or determines that the measured time difference cannot be accommodated by only manipulating the deep sleep stage of cycle m, the manipulation signal generation unit may further generate a manipulation signal for manipulating a previous sleep cycle, for example the cycle m-1. The condition used by the manipulation signal generation unit to decide whether one or more sleep cycles and/or sleep stages have to be manipulated relates to how long a deep sleep stage (or another sleep stage) can be extended or shortened. This condition may depend on the sleeping cycles to be manipulated. For example, at the beginning of a user's sleep, sleep stages may generally be manipulated for a longer time than towards the end of the user's sleep. Furthermore, this condition may depend on the user data.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. A device (100) for generating a manipulation signal for manipulating a user's sleep state, the device comprising:
- a sensor data input (4) configured to obtain sleep-related data (2) of a first user and a second user,
- an analysis unit (6) configured to determine one or more sleep parameters (8) of the first user and the second user based on the sleep-related data (2), and
- a manipulation signal generation unit (10) configured to generate a manipulation signal (12) for manipulating a sleep state of at least the second user based on the determined one or more sleep parameters (8) of the first user and the second user.

2. The device (100) as claimed in claim 1,
wherein the sleep-related data (2) comprises information about a current and/or one or more prior sleep parameters of the first user and the second user.

3. The device (100) as claimed in claims 1 or 2,
wherein the analysis unit (6) is configured to estimate a current sleep state and/or one or more prior sleep states and/or to predict one or more upcoming sleep states.

4. The device (100) as claimed in any of the preceding,
wherein the manipulation signal generation unit (10) is configured to generate the manipulation signal (12) based on a target condition of the first and/or the second user.

5. The device (100) as claimed in claim 4,
wherein the target condition comprises any of a target wake-up time, a target wake-up time range, particularly between a first wake-up time of the first user and a second wake-up time of the second user, a target sleep state and a target environmental condition, particularly a target illumination, a target temperature, a target air composition, a target smell, a target humidity, a target contact stimulation, a target vibration stimulation and a target sound.

6. The device (100) as claimed in claims 4 or 5,
wherein the manipulation signal generation unit (10) is configured to adjust the target condition of at least the second user based on the sleep-related data (2) and/or the determined one or more sleep parameters (8) of the first user and/or the second user.

7. The device (100) as claimed in any of the preceding claims,
wherein the manipulation signal generation unit (10) is configured to generate the manipulation signal (12) comprising synchronization information for synchronizing at least one sleep state of the second user with at least one sleep state of the first user.

8. The device (100) as claimed in any of the preceding claims,
wherein the manipulation signal generation unit (10) is configured to generate the manipulation signal comprising stimulation information for stimulating at least the second user.

9. The device (100) as claimed in claim 8,
wherein the stimulation information comprises information about changing any of temperature, air composition, smell, illumination, sound, humidity, a contact stimulation and a vibration stimulation.

10. The device (100) as claimed in any of the preceding claims,
wherein the manipulation signal generation unit (10) is configured to generate the manipulation signal (12) comprising any of shifting information for shifting a sleep state, shortening information for shortening a sleep state, extending information for extending a sleep state and transformation information for transforming a sleep state, in particular into a light sleep state or a deep sleep state.

11. The device (100) as claimed in any of the preceding claims,
wherein the analysis unit (6) is configured to determine the one or more sleep parameters (8) of the first user and/or the second user based on environmental data and/or user data.

12. A system (200) for manipulating a user's sleep state, the system comprising:
- a tracking unit (16) configured to acquire sleep-related data (2) of a first user and a second user by tracking sensor data (14) of the first user and the second user,
- a manipulation unit (22) configured to obtain a manipulation signal (12) and to manipulate a sleep state of at least the second user based on the manipulation signal, and
- a device (100) for generating a manipulation signal for manipulating a user's sleep state as claimed in claim 1.

13. The system (200) as claimed in claim 12,
wherein the tracking unit (16) comprises any of an electroencephalograph, an electrooculograph, an electromyograph, a camera, a movement sensor, an inertia sensor, a heart rate monitor, a heart rate variability monitor, a sweat rate monitor, a galvanic skin response sensor, a microphone, a pressure sensor, a respiration rate and respiration depth monitor, and a body temperature sensor.

14. A method for generating a manipulation signal for manipulating a user's sleep state, the method comprising:
- obtaining sleep-related data of a first user and a second user,
- determining one or more sleep parameters of the first user and the second user based on the sleep-related data, and
- generating a manipulation signal for manipulating a sleep state of at least the second user based on the determined one or more sleep parameters of the first user and the second user.

15. A computer program comprising program code means for causing a computer to carry out the steps of the method as claimed in claim 14 when said computer program is carried out on the computer.
